(19) Europäisches Patentamt
European Patent Office
Office européen des brevets

(11) Publication number: **0 051 442**
**A1**

(12) # EUROPEAN PATENT APPLICATION

(21) Application number: 81305101.8

(22) Date of filing: 28.10.81

(51) Int. Cl.$^3$: C 07 D 207/327

(30) Priority: 03.11.80 US 203052
24.11.80 US 209950

(43) Date of publication of application:
12.05.82 Bulletin 82/19

(84) Designated Contracting States:
BE CH DE FR GB IT LI NL SE

(71) Applicant: ETHYL CORPORATION
330 South Fourth Street
Richmond Virginia(US)

(72) Inventor: Stahly, Glenn Patrick
6449 Peggy Street
Baton Rouge Louisiana(US)

(74) Representative: Baillie, Iain Cameron et al,
c/o Ladas & Parry Isartorplatz 5
D-8000 München 2(DE)

(54) **Process for producing substituted pyrroles.**

(57) A process for the preparation of a lower alkyl pyrrole-2-acetic acid ester by reaction of a pyrrole reactant with a mixture containing an acid, a lower alkanol and water, the product being recovered and subsequently used to prepare valuable anti-inflammatory agents.

EP 0 051 442 A1

# PROCESS FOR PRODUCING SUBSTITUTED PYRROLES

This invention relates to a process for producing 5-aroyl-pyrroles and, more particularly, to 1,4-dialkyl-5-aroyl-pyrrole alkanoic acids and the corresponding salts, esters, nitriles, amides and substituted amides thereof.

Such 5-aroyl-pyrroles are known as effective anti-inflammatory compounds having useful pharmacological properties and which are suitable for incorporation into conventional pharmaceutical forms for administration. The anti-inflammatory activity has been demonstrated in the standard kaolin-induced rat paw edema and cotton pellet granuloma tests at doses generally ranging from 5-100 mg/kg body weight. The compounds produced by the improved process of the present invention can be more particularly illustrated by reference to prior art patents in this particular art.

Specifically, Carson U.S. 3,752,826 issued August 14, 1973, teaches a number of compounds in the class of 5-aroyl-pyrrole alkanoic acids and the corresponding acid derivatives thereof which are useful as anti-inflammatory agents and as synthetic intermediates. Particularly, Carson teaches the compounds *per se* and methods for their preparation.

U. S. 3,865,840, issued February 11, 1975, and 3,952,012, issued April 20, 1976, are divisions of Carson's U. S. 3,752,826 and are particularly directed to the process of making loweralkyl 1,4-diloweralkyl-3-lower alkoxy carbonyl-pyrrole-2-acetate compounds and a process of making a loweralkyl 5-aroyl-1-$R_4$-4-$R_5$-pyrrole-2-acetate in

which $R_4$ and $R_5$ represent lower alkyl compounds. The Carson '840 patent teaches a process of reacting, at just below 60°C. a mixture of a loweralkyl amine of the formula $R_4-NH_2$ and a dilower alkyl acetone dicarboxylate of the formula:

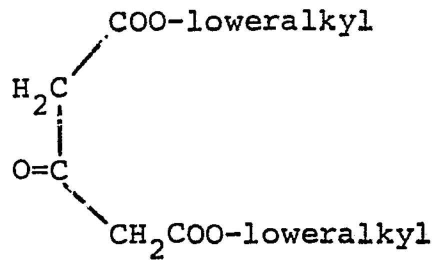

with a chloromethyl loweralkyl ketone of the formula: $Cl-CH_2-CO-R_5$, wherein the foregoing formulas said $R_4$ and said $R_5$ represent loweralkyl. The resulting pyrrole diester is then employed according to the teaching of the Carson '012 patent to produce the loweralkyl 5-aroyl-1-$R_4$-4-$R_5$-pyrrole-2-acetate by the steps of (1) hydrolyzing the diester under alkaline conditions to give a diacid, (2) partially re-esterifying with an acidic solution of a lower alkanol to give a loweralkyl 1-$R_4$-4-$R_5$-3-carboxy-pyrrole-2-acetate, (3) decarboxylating the 3-carboxy group to form a pyrrole ester by heating the partially re-esterified pyrrole to carbon dioxide elimination temperatures and (4) acylating the lower 1-$R_4$-4-$R_5$-pyrrole-2-acetate with an aroyl chloride in the presence of a Lewis acid in an organic solvent suitable for Friedel-Crafts acylation reactions to obtain the desired loweralkyl 5-aroyl-1-$R_4$-4-$R_5$-pyrrole-2-acetate.

The process for preparation of such pyrroles is more difficult because placing the alkyl substituent in the 4-position is extremely difficult after the pyrrole ring is formed. This is especially true when the ring contains other substituents. Accordingly, the '012 patent teaches that it is necessary for the formation of the 4-loweralkyl-substituted 5-aroyl-pyrroles to proceed through the laborious sequence of ring formation producing the diester, hydrolysis of the diester to the diacid,

partial re-esterification to the 2-acetate, decarboxylation of the 3-carboxy group and then hydrolysis to the acid. In an alternate procedure the acylation to the 5-aroyl-pyrrole diester can take place after ring formation of the pyrrole diester, followed by the above-described sequence of hydrolysis, partial re-esterification, decarboxylation and hydrolysis.

Skilled artisans, of course, realize that the value of intermediates in a process increases with each process step performed to synthesize the desired end product. Thus, elimination of one or more process steps while accomplishing the same goals is a valuable and often economic process improvement which can be used to great advantage. The advantages of such simplifications include elimination of handling steps with their attendant losses of increasingly valuable intermediates, increase of positive process control, decrease of opportunity for introduction of impurities, decrease in waste stream handling, decrease in processing costs for utilities to move large amounts of intermediate materials from one reactor to the other and usually, but not always, increase in yield.

This invention is based on the discovery that several steps in the prior art process for the preparation of 1,4-dilower-alkyl-5-aroyl-pyrrole-2-acetic acids and its corresponding salts, esters, nitriles, amides and substituted amides can be eliminated.

The present invention provides a process for preparing a loweralkyl 1-$R_4$-4-$R_5$-pyrrole-2-acetate of the formula:

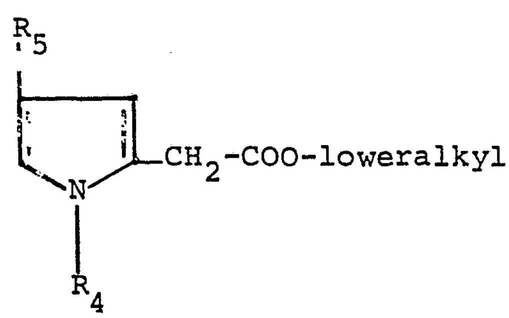

which comprises contacting a pyrrole reactant compound of formula:

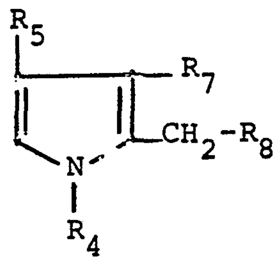

with a mixture of strong mineral acid, a lower alkanol and water, wherein the foregoing formulas said $R_4$ and said $R_5$ each are independently selected from loweralkyl groups, and said $R_7$ and said $R_8$ each are independently selected from COOH, COO-loweralkyl, and COOM in which said M is an alkali metal selected from sodium and potassium.

The present process preferably employs a pyrrole diester from the ring-formation step in a process for producing 5-aroyl-pyrrole alkanoic acids, or any suitable pyrrole reactant compound having a carboxylic acid, carboxylate ester or carboxylate salt group substituted at the 2- or 3-position, such as 3-alkoxy-carbonylpyrrole-2-acetate, a 3-carboxypyrrole-2-acetate, a 3-carboxypyrrole-2-acetic acid, a 3-carboxypyrrole-2-acetic acid alkali metal salt, a 3-alkoxycarbonylpyrrole-2-acetic acid alkali metal salt, a 3-carboxypyrrole-2-acetic acid dialkali metal salt and the like.

Preferably, the present process employs a lower alkyl pyrrole diester. More preferably, the pyrrole starting diester is a precursor for valuable pharmaceutical products, as indicated in the prior art hereinabove cited. Thus, the starting pyrrole diester and, as well, the other pyrrole reactant compounds include a pyrrole ring having the nitrogen atom substituted with a lower alkyl group which can be methyl, ethyl, propyl or butyl. Similarly, the 4-position of the pyrrole ring is substituted with a lower alkyl group. The pyrrole ring is substituted in the 2-position with an alkylene chain bearing an ester group. The alkylene chain can be one or more carbon atoms. Thus, the ester group in the 2-position can be an acetate, a propionate, or butyrate group, although the acetate group

is preferred. The other ester group is attached at the 3-position and the carbonyl carbon atom is directly attached to the pyrrole ring. Both ester groups can have, as the alcohol portion thereof, a lower alkyl group such as methyl, ethyl, propyl, or butyl. The 5-position can be unsubstituted or substituted with an aroyl group of the character recited in the prior art patents. Specifically, if the pyrrole ring is substituted in the 5-position, the aryl group which is connected to the pyrrole ring by a carbonyl carbon atom is a member selected from the group consisting of phenyl, thienyl, 5-methyl-thienyl, monosubstituted phenyl and polysubstituted phenyl, each substituent of the substituted phenyl groups being a member selected from the group consisting of halo, loweralkyl, trifluoromethyl, loweralkoxy, nitro, amino, cyano and methylthio. In order not to lose valuable and expensive reagents, it is generally considered desirable to have the pyrrole diester, or other pyrrole reactant compound, unsubstituted in the 5-position until it is converted to the monoester. The pyrrole diester starting material may be employed in any convenient form. For example, it may be in its pure form as a white solid or it may be in a solution or crude mixture with a liquid carried over from the process in which it is produced.

The pyrrole diester or other pyrrole reactant compound is reacted with a mixture containing an acid, water, and a lower alkanol. In general, the acid is a strong acid and preferably a mineral acid. Typical of useful acids are sulfuric acid, phosphoric acid, and perchloric acid. Any acid which catalyzes the selective ester cleavage of the pyrrole diester, but does not degrade the product pyrrole monoester, is suitable. In general, the acid should be highly concentrated; that is, the acid should not be diluted with water beyond the amount suitable for the proper ratio of acid, water, and alkanol. Thus, highly dilute acids are undesirable because the dilute acids react with and degrade the product of the present process.

In contrast, concentrated acids do not rapidly affect the product pyrrole monoester and are thus more highly preferred. Specifically, it has been found that concentrated sulfuric acid containing 96% sulfuric acid and the remainder water is a highly preferred acid for use in this process.

The reagent mixture includes water because, although large amounts of water result in product degradation from dilute acid, it has also been found that a reagent mixture containing very low amounts of water gives unsuitable yields. Thus, a small amount of water is preferred.

As a final constituent of the reagent mixture, there is employed an alkanol, preferably one which is compatible with the ester groups. It has been found that without an alcohol present the yields of desired product are decreased. Typical of the alkanols which can be employed are methanol, ethanol, propanol, isopropanol and butanol. From the above, it is seen that preferred alkanols employed in this invention are lower alkanols having from 1 to 4 carbon atoms. Of course, when the alcohol portion of the ester group is the ethyl ester it is preferred to employ ethanol. Similarly, a propyl or methyl ester would more preferably employ propanol or methanol, respectively. Accordingly, it is more highly preferred to employ a lower alcohol having the same number of carbon atoms in the chain used in the ester group.

The nature and proportional amount of reagents in the reagent mixture is highly important. It has been found that an increase in the amount of acid relative to the amount of water increases the yield. However, this reaches a maximum and further increase in the acid concentration, or decrease in the water concentration, will result in a decrease in yield. Preferred molar ratios of acid to water range from 0.3:1 to 10:1. Similarly, the molar ratio of acid to alkanol can be varied so that an increase in the concentration of acid with respect to alkanol will provide increased yields up to a point after which further increased acid to alkanol concentrations

cause decreased yields. In general, the molar ratio of acid:alkanol is preferably from 0.5:1 to 6:1. The amount of acid employed can range from 15 to 2 mols of acid per mol of starting pyrrole. More preferably, a ratio of 10 to 3.75 has been found to be quite effective.

Having generally established the relationship of the reagent mixture, it should be pointed out that the optimum ratio is not necessarily limited to the specific values within the stated limits because one reagent will have an affect on the other. Thus, a high ratio of acid to starting pyrrole reactant may indicate a correspondingly high amount of lower alkanol while a relatively low ratio of acid may indicate a relatively lower amount of alkanol. In any case, it is desirable to minimize the amounts of reagents employed and to optimize the ratio of one reagent to the other in order to simplify processing, maintaining high yield, decrease the amount of impurities formed, and decrease reagent purification, disposal or recycle. Therefore, the values of a particular reagent within the ratios above stated will be subject to variation as one skilled in the art will be aware.

A most highly preferred pyrrole reactant is the 3-alkoxycarbonylpyrrole-2-acetate. From experiments observed, it has been determined that a highly preferred molar ratio for this pyrrole reactant will range from about 5:1 to 2:1 moles of acid per mole of water and from about 1:1 to 5:1 moles of acid per mole of alkanol. Another highly preferred pyrrole reactant is a 3-carboxy-pyrrole-2-acetic acid. Experimental work indicates that highly preferred molar ratios of acid to water for this pyrrole reactant compound range from 2:1 to 5:1 moles of acid per mole of water. Also, the molar ratios of acid to alkanol which are highly preferred for this reactant range from 0.75:1 to 2:1 moles of acid per mole of alkanol.

The process of the present invention can be carried out at elevated temperatures. However, the temperature should not be so high as to degrade the products.

In general, as the temperature increases, an increase in the rate of the reaction is observed. Temperatures from 40°C. up to 100°C. have been found to be useful. However, decarboxylation temperatures of 200-210°C. which were employed in prior art processes, have been found to adversely affect the product and substantially reduce the yields. Preferably, the temperature should be maintained at between 60 and 85°C. depending on the length of time the reaction is to be run. The time of reaction is likewise not the most critical variable and reaction can be maintained for over 60 hours at 60°C. with little product loss. However, reaction times as short as five hours have been found useful and, in small scale experiments, the reaction is usually complete in less than one hour after the reaction mixture is brought to a temperature of 80°C. Accordingly, temperature and reaction time are not strictly independent variables but can be adjusted to suit the requirements of the process.

The pyrrole reactant compound, preferably the pyrrole diester, can be placed in the reaction vessel and the reagent mixture added thereto. In many instances, however, it may be desirable to add the pyrrole reactant to the reagent mixture. The reagent mixture can be premixed and added to the pyrrole reactant or, if the reagents are added separately, the lower alkanol can be added, followed by the acid and the water. Alternatively, aqueous acid may be used so that the water for the reagent mixture may be contained therein, provided it does not exceed the above-described molar ratios. After the reaction mixture is in the vessel, it is heated with stirring for a period sufficient to complete the reaction.

The product can be recovered by extraction into an organic phase. Recovery of the product should be carried out so that handling losses are minimized and degradation by-products are not produced. In one recovery method, an organic solvent is added to the cooled reaction mixture and the cool reaction mixture is diluted with cold

water while using vigorous agitation. The monoester product is more soluble in the concentrated acid than in the organic phase; however, on dilution of the acid with water, the product monoester dissolves rapidly into the organic phase. However, as noted above, it has been found that dilute acid degrades the product pyrrole monoester and it is therefore preferable that the reaction mixture be held at a relatively low temperature during dissolution or extraction in order to minimize product loss. Organic solvents useful in recovery of the product are those in which the pyrrole monoester product is soluble to a much greater extent than the dilute acid. Accordingly, chlorinated hydrocarbon solvents and aromatic hydrocarbon solvents are particularly useful. Specifically, methylene chloride, carbon tetrachloride, chloroform, dichloroethane, benzene, toluene, xylene, monochlorobenzene and the like are useful solvents. In order to avoid unnecessary solvent recovery and purification steps in the present process when practiced on a commercial scale, it has been found that solvents useful in subsequent steps for preparation of the anti-inflammatory pharmaceutical compounds can be more profitably employed in the present process. One such solvent is toluene which is highly preferred.

The product pyrrole monoester is then recovered by stripping the solvent from the product or the product can be carried on for further processing according to the procedures described in the prior art.

Having described the process in general, it is believed that the following examples will illustrate further specific embodiments of the process of the present invention. In some examples, comparative experiments are shown to illustrate the necessity for certain reagents and the presence or absence of certain reagents and the variability of conditions are shown to illustrate the effect thereon of the process.

EXAMPLE 1

To 50 grams of a solution of 20% ethyl 1,4-dimethyl-3-ethoxycarbonylpyrrole-2-acetate (10 grams, 40 mmoles) in toluene containing 2.4 ml of absolute ethanol (1.9 grams, 41 mmoles) was added, dropwise, over 20 minutes, 8.6 ml of 96% sulfuric acid (15 grams, 160 mmoles, containing 0.63 gram, 35 mmoles of water). During the addition, the temperature of the reaction mixture rose to about 50°C. and the mixture became heteregeneous. The reaction mixture was then heated in an oil bath at 75-80°C. for 75 minutes with stirring. Gas evolution was observed. The reaction mixture was then diluted with 25 ml of toluene and cooled to 5°C. Then 75 ml of ice water was added in one portion with vigorous stirring. After five minutes, the organic layer was separated and dried with magnesium sulfate. Gas chromatographic analysis indicates the toluene contained 9% ethyl 1,4-dimethylpyrrole-2-acetate. The toluene was removed by vacuum distillation at 100 mm of mercury followed by vacuum distillation of the residue at 1 mm of mercury to give a fraction which weighed 5.5 grams. This corresponds to a 78% yield of ethyl 1,4-dimethylpyrrole-2-acetate.

Gas chromatographic analysis employed a Hewlett-Packard Model 5830A instrument using a 10 foot, 1/8 inch column packed with 10% DEGS on Chromosorb Q. Dibutyl phthalate was used as an internal standard and was added to the sample after work-up to avoid decomposition in the strong acid.

EXAMPLES 2-8

The purpose of this series of experiments is to illustrate the requirement for each of the components in the reagent mixture and to establish relative ratios of sulfuric acid, ethanol and water. To a number of 100 mg samples of ethyl 1,4-dimethyl-3-ethoxycarbonylpyrrole-2-acetate was added a variety of different solutions. The resultant mixtures were heated for about 18 hours at 65-66°C. followed by pouring into 10 ml of water and extracting

three times with 10 ml of methylene chloride. Each organic solution was dried with magnesium sulfate and the solvent was removed *in vacuo*. The residues were then heated at 190-200°C. for 20 minutes to assure that they were entirely decarboxylated. Since all samples were treated in the same manner and the only variation was with respect to the reagent mixture added, the examples illustrate the relative importance of the components in that mixture. The composition of the reagent mixture and the results obtained as percent yield of by-products, starting materials and desired ethyl 1,4-dimethylpyrrole-2-acetate are given in the following Table 1.

TABLE 1

Reagent Mixture Composition Variability Study

| Example No. | Reagent Composition (Molar Ratio) $H_2SO_4/EtOH/H_2O$ | % Yield* By-Products | Pat** | PDE** |
|---|---|---|---|---|
| 2 | 1/0/0.22 | 21 | 3 | <1 |
| 3 | 1/0.17/0.22 | 15 | 8 | 16 |
| 4 | 1/0/0.77 | 13 | 7 | 2 |
| 5 | 1/4.8/16 | 26 | nd | 38 |
| 6 | 1/1.9/6.4 | 18 | 19 | 25 |
| 7 | 1/0.47/1.8 | 9 | 48 | nd |
| 8 | 1/0.24/0.50 | 15 | 26 | 13 |

nd = none detected

* Yield determined by gas chromatography using an internal standard.

** PAT - Ethyl 1,4-dimethylpyrrole-2-acetate
PDE - Ethyl 1,4-dimethyl-3-ethoxycarbonylpyrrole-2-acetate

Note that the absence of ethanol and the low amount of water in Examples 2 and 4 and the low amount of ethanol in Example 3 provides very low yields of the desired product. In contrast, when both ethanol and water in sufficient relative proportions are included, such as in Example 7, the yield of desired product is much higher. Further, Examples 2-8 show that the relative ratio of acid to ethanol to water is important since if too much ethanol

and water are present, the yield is substantially lower or no reaction occurs. However, as the ratio of acid to both ethanol and water increases, the yield of desired product reaches a maximum and then begins to decrease as the amount of acid exceeds by a large amount the relative amounts of ethanol and water, note Examples 5 to 8, particularly. Therefore, the above results indicate that acid, alkanol and water are all required and that the relative proportions of each of the reagents is of great significance.

The effects of temperature and time were determined for a specified reagent mixture of sulfuric acid, ethanol and water at a molar ratio of 1:0.47:1.8, respectively, in the following Examples 9-10. Two constant temperature runs were made in which 0.5 gram of ethyl 1,4-dimethyl-3-ethoxycarbonylpyrrole-2-acetate was put in 6.7 grams (about 5 ml) of a solution consisting of 96% sulfuric acid, water and absolute ethanol in a volume ratio of 2:1:1. The reaction mixture was heated at 60° in Example 9 and at 80° in Example 10 under a water condenser. Every hour, 1/2 ml of reaction mixture was worked up by pouring into 5 ml of water and extracting three times with 5 ml portions of methylene chloride. The organic layers were dried with magnesium sulfate, stripped and the resultant residue was analyzed by gas chromatography using dibutyl phthalate as an internal standard. The results of the two examples are shown in Table 2 below.

TABLE 2

Effect of Temperature and Time on Preparation of Ethyl 1,4-Dimethylpyrrole-2-Acetate

| Example No. | Temp. (°C) | Reaction Time (Hr) | Percent Yield+ TMP* | PAT* | TMPE* | PDE* |
|---|---|---|---|---|---|---|
| 9 | 60 | 1 | 10 | 27 | 9 | 30 |
| | | 2 | 18 | 48 | 3 | 15 |
| | | 3 | 22 | 59 | <1 | 7 |
| | | 4 | 23 | 62 | <1 | 3 |
| | | 5 | 23 | 62 | nd | 1 |
| | | 6 | 26 | 65 | " | nd |
| | | 24 | 24 | 67 | " | " |
| 10 | 80 | 1 | 28 | 61 | nd | nd |
| | | 2 | 30 | 57 | " | " |
| | | 3 | 30 | 58 | " | " |
| | | 4 | 33 | 57 | " | " |
| | | 5 | 29 | 48 | " | " |
| | | 6 | 37 | 47 | " | " |
| | | 72 | 26 | 28 | " | " |

nd = none detected

reagent used - 2/1/1 volume mixture of 96% $H_2SO_4$/ethanol/$H_2O$

* TMP - 1,2,4-trimethylpyrrole

PAT - Ethyl 1,4-dimethylpyrrole-2-acetate

TMPE - 1,2,4-trimethyl-3-ethoxycarbonylpyrrole

PDE - Ethyl 1,4-dimethyl-3-ethoxycarbonylpyrrole-2-acetate

\+ Percent yield determined by gas chromatography using an internal standard.

From the table, it can be seen that the reaction time and temperature are not as critical as the reagent composition. Similar yields of the desired product, ethyl 1,4-dimethylpyrrole-2-acetate, were obtained after 3 or 4 hours at 60° or after 1 hour at 80°.

EXAMPLES 11-24

The following series of examples illustrate the effect of varying the pyrrole diester treating reagent composition by using different relative amounts of acid, lower alkanol and water. The effect on the yield of the pyrrole monoester is clearly shown.

In a suitable flask with a water condenser, the ethyl 1,4-dimethyl-3-ethoxycarbonylpyrrole-2-acetate was heated with a reagent solution of a specified composition, all as given in the following table, in an oil bath for one hour at a temperature of 76-84°C. After the resultant reaction mixture was allowed tc cool, it was poured into 10 ml of water and extracted three times with 10 ml portions of methylene chloride. The combined organic layers were dried with magnesium sulfate, concentrated *in vacuo* and the residue was subjected to gas chromatographic analysis using dibutyl phthalate as the internal standard. The results of these runs are given below in Table 3.

TABLE 3

Preparation of Ethyl 1,4-Dimethylpyrrole-2-Acetate Using Variable Reagent Composition

| Example No. | Amount of PDE (mg) | Amount (g) | Composition $H_2SO_4$/EtOH*/$H_2O$ (Molar Ratio) | Percent Yield+ TMP* | PAT* | TMPE* | PDE* |
|---|---|---|---|---|---|---|---|
| 11 | 100 | 1.862 | 1/0.11/0.61 | 14 | 7 | 14 | 8 |
| 12 | 101.6 | 1.6593 | 1/0.19/0.89 | 13 | 15 | 15 | 20 |
| 13 | 98.7 | 1.6023 | 1/0.31/1.3 | 28 | 55 | <1 | 3 |
| 14 | 102.6 | 1.2917 | 1/0.47/1.8 | 24 | 61 | nd | <1 |
| 15 | 100 | 1.2749 | 1/0.72/2.6 | 22 | 66 | " | 2 |
| 16 | 103.1 | 1.2271 | 1/1.1/3.9 | 9 | 16 | 16 | 39 |
| 17 | 101.3 | 1.0631 | 1/1.9/6.4 | <1 | <1 | 18 | 55 |
| 18 | 102.4 | 1.325 | 1/0.86/0.53 | 10 | 78 | nd | 2 |
| 19 | 104.1 | 1.392 | 1/0.75/0.83 | 15 | 74 | " | 2 |
| 20 | 101.4 | 1.4187 | 1/0.67/1.2 | 14 | 64 | " | 1 |
| 21 | 101.8 | 1.4974 | 1/0.58/1.5 | 17 | 49 | 3 | 12 |
| 22 | 101.4 | 1.3070 | 1/0.39/2.1 | 26 | 48 | 1 | 7 |
| 23 | 100.8 | 1.4261 | 1/0.28/2.4 | 39 | 47 | nd | nd |
| 24 | 100.5 | 1.317 | 1/0.19/2.7 | 46 | 40 | " | <1 |

nd = none detected

*PDE - Ethyl 1,4-dimethyl-3-ethoxycarbonylpyrrole-2-acetate

TMP - 1,2,4-trimethylpyrrole

PAT - Ethyl 1,4-dimethylpyrrole-2-acetate

TMPE - 1,2,4-trimethyl-3-ethoxycarbonylpyrrole

EtOH - Ethanol

+ Percent yield determined by gas chromatography using an internal standard.

For the purposes of illustration, the strong acid employed in the previous examples has been sulfuric acid. The following examples illustrate the use of other strong acids which are illustrative of the invention.

EXAMPLE 25

To 101 mg of ethyl 1,4-dimethyl-3-ethoxycarbonylpyrrole-2-acetate was added 1 ml of 1:1 volume mixture of absolute ethanol and 86% phosphoric acid. The mixture was heated in an 80°C. oil bath for 1 hour, poured into 10 ml of water, and extracted with three 10 ml portions of

methylene chloride. The combined organic layers were dried (magnesium sulfate) and concentrated *in vacuo* to give a residue weighing 67 mg. Gas chromatographic analysis of this residue indicated it contained 33 mg (46%) of ethyl 1,4-dimethylpyrrole-2-acetate.

EXAMPLE 26

To 103 mg of ethyl 1,4-dimethyl-3-ethoxycarbonyl-pyrrole-2-acetate was added 1 ml of a solution of 1.5 ml of absolute ethanol in 5 ml of 70% perchloric acid. The mixture was heated in an 80°C. oil bath for 1 hour, poured into 10 ml of water, and extracted with three 10 ml portions of methylene chloride. The combined organic layers were dried (magnesium sulfate) and concentrated *in vacuo* to give a residue weighing 69 mg. Gas chromatographic analysis of this residue indicated it contained 49 mg (66%) of ethyl 1,4-dimethylpyrrole--2-acetate.

The pyrrole monoester product can be prepared from the diester neat or in the presence of a solvent. Further, while the work-up is not critical, it can have an effect on the yield of the product because it has been found that the dilute acid will degrade the product pyrrole monoester. Accordingly, the following examples illustrate preparation of the product pyrrole monoester in the presence of a solvent and show the results of work-up under conditions which do not adversely affect product yields.

EXAMPLES 27-44

The indicated weight of starting material was reacted with the reagent mixture indicated in Table 4 below. The starting material was initially in methylene chloride or toluene solution or without any solvent. After reaction at 80° for 75 minutes, each reaction mixture was cooled to room temperature (the reaction mixture was cooled to about 5°C. in Example 44), diluted with water as indicated, and extracted the stated number of times with the amount of extracting solvent given. Yields are based on either gas chromatographic analysis or on the product weight after distillation, as indicated.

TABLE 4

Preparation of Ethyl 1,4-Dimethylpyrrole-2-Acetate

| Example No. | PDE[b] | Moles Used per Mole of PDE[b]: $H_2SO_4$ | EtOH | $H_2O$ | $H_2O$ Used in Dilution (Moles per Mole PDE[b]) | Extracting Solvent | Solvent Used per Extract (Moles per Mole PDE[b]) | Number of Extracts | % Yield[c] PAT[b] |
|---|---|---|---|---|---|---|---|---|---|
| 27 | T[a] | 5 | 1 | 1.1 | 105 | Toluene | 16 | 3 | 66 |
| 28 | T | 5 | 1 | 1.1 | 328 | $CH_2Cl_2$ | 66 | 3 | 65 |
| 29 | N | 5 | 1 | 1.1 | 328 | $CH_2Cl_2$ | 66 | 3 | 73 |
| 30 | N | 5 | 1 | 1.1 | 117 | Toluene | 16 | 3 | 63<br>60* |
| 31 | T | 5 | 1 | 1.1 | 117[d]<br>234[d] | Toluene<br>Toluene | 16<br>16 | 4<br>4 | 62<br>63 |
| 32 | T | 5 | 1 | 1.1 | 117 | Toluene | 16 | 4 | 62 |
| 33 | N | 10 | 10 | 5 | 328 | $CH_2Cl_2$ | 66 | 3 | 76 |
| 34 | N | 10 | 10 | 5 | 117 | Toluene | 16 | 4 | 64 |
| 35 | T | 10 | 10 | 5 | 84 | Toluene | 5 | 5 | 54* |
| 36 | N | 9 | 0.65 | 2 | 94 | Toluene | 16 | 3 | 15<br>11* |
| 37 | 2g pure | 10 | 10 | 5 | 105 | $CH_2Cl_2$ | 20 | 4 | 72* |
| 38 | 2g pure | 10 | 10 | 5 | 105 | Toluene | 20 | 4 | 67 |
| 39 | 3g pure | 10 | 10 | 5 | 105 | $CH_2Cl_2$[e]<br>Toluene | 40<br>40 | 4<br>4 | 76<br>74 |

TABLE 4 CONTINUED

Preparation of Ethyl 1,4-Dimethylpyrrole-2-Acetate

| Example No. | PDE[b] | Moles Used per Mole of PDE: $H_2SO_4$ | EtOH[b] | $H_2O$ | $H_2O$ Used in Dilution (Moles per Mole PDE[b]) | Extracting Solvent | Solvent Used per Extract (Moles per Mole PDE[b]) | Number of Extracts | % Yield[c] PAT[b] |
|---|---|---|---|---|---|---|---|---|---|
| 40 | 10g 63% melt | 5 | 1 | 1.1 | 223 (at room temp). | Toluene | 19 | 4 | 66* |
| | | | | | 223 (at 5°C)[d] | Toluene | 19 | 4 | 67* |
| 41 | 10g 63% melt + 2ml $CH_2Cl$ | 5 | 1 | 1.1 | 223 | $CH_2Cl_2$ | 19 | 4 | 73* |
| 42 | 50g 21% soln.in toluene | 5 | 1 | 1.1 | 134 | Toluene | 11 | 4 | 74* |
| 43 | 50g 21% soln.in toluene | 3.75 | 1 | 0.8 | 134 | Toluene | 11 | 4 | 67* |
| 44 | 50g 21% soln.in toluene | 3.75 | 1 | 0.8 | 100 | Toluene | 16 | 1 | 79* |

All reactions were heated at 80°C. for 75 min.

(a) N = neat, T = toluene solution.
(b) Same abbreviations as used previously in Tables 1, 2 and 3.
(c) Gas chromatographic analysis using internal standard; otherwise * indicates product isolated by distillation and yield calculated based on weight of isolated product.
(d) Reaction mixture divided in half.
(e) Reaction mixture divided in half.

From the above examples, it is clear that reactions taking place with the starting pyrrole diester in toluene solution produce yields which are as good or better than those using no solvent and that the use of a lower temperature water work-up is more favorable than work-up at room temperature or higher.

EXAMPLE 45

To 101.5 mg of ethyl 1,4-dimethyl-3-carboxypyrrole-2-acetate was added 0.13 ml of ethanol and, dropwise, 0.13 ml of 96% sulfuric acid. The reaction mixture was then heated at 80°C. for 1 hour and 15 minutes, cooled and poured into 2 ml of water. The diluted reaction mixture was then immediately extracted three times with 5 ml portions of methylene chloride. The combined organic layers were dried over magnesium sulfate and the methylene chloride was removed *in vacuo* to give 76.5 mg of liquid. This was shown to contain, by gas chromatographic analysis using an internal standard, 75% ethyl 1,4-dimethylpyrrole-2-acetate, which is a 70.4% yield.

EXAMPLE 46

To 99.8 mg of 1,4-dimethyl-3-ethoxycarbonylpyrrole-2-acetic acid was added 0.13 ml of ethanol and, dropwise, 0.13 ml of 96% sulfuric acid. The reaction mixture was then heated at 80°C. for 1 hour and 15 minutes, cooled and poured into 2 ml of water. The diluted reaction mixture was then immediately extracted three times with 5 ml portions of methylene chloride and the methylene chloride was removed *in vacuo* to give 76.1 mg of a liquid. This was shown to contain, by gas chromatographic analysis using an internal standard, 72% ethyl 1,4-dimethylpyrrole-2-acetate, which is a 68.25% yield.

EXAMPLE 47

To a mixture of 6.13 g (0.06 mole) of 96% $H_2SO_4$ and 2.07 g (0.045 mole) of ethanol, cooled to room temperature was added 3.69 g (0.015 mole) of 80% by weight 1,4-dimethyl-3-carboxypyrrole-2—acetic acid. This provided an initial molar ratio of 1:4:0.5:3 of pyrrole reactant to

acid to water to alkanol, respectively. The reaction mixture was heated with agitation at 70°C. for 3.5 hours; after 30 minutes of heating 7.5 ml of toluene was added to aid agitation and decrease foaming caused by gas evolution. The reaction mixture was cooled to room temperature, 16 g toluene was added and then a solution of 2.40 g of NaOH (0.06 moles) and 10.8 g $H_2O$ was slowly added at ice bath temperatures. The reaction mixture was stirred for 30 minutes and then the organic and aqueous phases were separated. Gas chromatographic analysis of the toluene solution indicated 6.44% of ethyl 1,4-dimethylpyrrole-2-acetate, which is a 59.4% yield.

EXAMPLE 48

Following the procedure of Example 47, another experiment was carried out in the same manner except that 3.46 g (0.075 moles) of ethanol were employed to give a molar ratio in the initial reaction mixture of 1:4:0.5:5 of pyrrole reactant, acid, water and alkanol, respectively. After the same heating procedure as above, followed by cooling to room temperature, toluene and caustic work-up and separation, there was obtained 25.44 g of the organic phase, which by gas chromatographic analysis, contained 6.58% ethyl 1,4-dimethylpyrrole-2-acetate, which is a 61.7% yield.

EXAMPLE 49

The procedure of Examples 47 and 48 was varied by mixing all reactants except the sulfuric acid and then adding it gradually over a period of time. To an agitated mixture of 3.46 g (0.075 mole) of ethanol, 2.90 g (0.015 mole) of 1,4-dimethyl-3-carboxypyrrole-2-acetic acid and 7.5 g of toluene was added 6.13 g (0.06 mole) of 96% sulfuric acid, dropwise, over a period of 30 minutes. The reaction mixture was heated at 70°C. for 2 hours and then cooled to room temperature. Then 16 g toluene was added, the reaction mixture was placed in ice water cooling and a solution of 2.4 g (0.06 mole) sodium hydroxide and 12.8 g water was added over ten minutes. The resultant

mixture was stirred for 30 minutes and the organic phase, weighing 26.07 g, was separated. Based on the gas chromatographic analysis, the yield of ethyl 1,4-dimethylpyrrole-2-acetate was 79.8%.

EXAMPLE 50

The procedure of Example 49 was repeated except that the initial reactant mixture was heated in a 60°C. bath for 30 minutes followed by increasing the bath temperature to 80°C. and heating for an additional 1 hour and 40 minutes. Following work-up, as previously described, there was obtained 26.05 g of the organic layer, which is an 83.6% yield of ethyl 1,4-dimethylpyrrole-2-acetate.

EXAMPLE 51

To a stirred mixture of 2.56 g (0.0117 mole) of 1,4-dimethyl-3-carboxypyrrole-2-acetate monosodium salt and 2.7 g (0.0585 mole) of ethanol in 10 g of toluene at room temperature slowly was added 4.78 g (0.0468 mole) of 96% sulfuric acid over a fifteen minute period. The initial molar ratio of pyrrole reactant to acid to water to alkanol was 1:4:0.9:5, respectively. The reaction mixture was heated for 30 minutes in a 60°C. bath and then for one hour and 15 minutes in an 80°C. bath. The reaction mixture was then cooled to room temperature and 8.3 g of toluene were added. The reaction mixture was then placed in an ice water bath and a solution of 1.9 g of NaOH and 8.5 g of water was slowly added. After stirring at room temperature for thirty minutes, another 2.1 g of water was added to dissolve solids which appeared. The phases were separated and the organic phase weighed 20.22 grams. Gas chromatographic analysis showed 7.34% ethyl 1,4-dimethylpyrrole-2-acetate, which is a 70.1% yield. The analysis also showed 1.83% of ethyl 1,4-dimethyl-3-ethoxycarbonylpyrrole-2-acetate which is a 12.5% yield.

EXAMPLE 52

The procedure of Example 51 was followed, except that the pyrrole reactant was 2.3 g (0.0095 mole) of 1,4-dimethyl-3-carboxypyrrole-2-acetic acid disodium salt.

This was mixed with 2.2 g (0.0478 mole) of ethanol and 14.9 g (0.0162 mole) of toluene using stirring at room temperature. Then 5.85 g (0.0573 mole) of 96% sulfuric acid was added. The molar ratio of pyrrole reactant to acid to water to alkanol was 1:6:1.3:5, respectively. After stirring for 30 minutes upon completion of the mixture, the reaction mixture was heated in a 60°C. bath for 30 minutes and then placed in a 80°C. bath for 2 hours. The reaction mixture was then cooled with an ice water bath and a solution of 1.55 g of NaOH and 6.9 g of water was slowly added over a fifteen minute period. The diluted reaction mixture was stirred for 30 minutes while being brought to room temperature and the organic phase, weighing 16.38 g, was separated. Gas chromatographic analysis shows the organic phase contained 8.74% of ethyl 1,4-dimethylpyrrole-2-acetate and 0.644% of ethyl 1,4-dimethyl-3-ethoxycarbonylpyrrole-2-acetate, which are yields of 83.3% and 4.4%, respectively.

Although the examples have been given illustrating Applicant's invention with sulfuric acid, ethanol and water as the reagent composition, one skilled in the art can plainly see from the other examples with phosphoric acid and perchloric acid that other reagents can be employed. Similarly, other alkanols can be used and the optimum reagent compositions must be determined following the procedures outlined hereinabove.

CLAIMS

1. A process for preparing a loweralkyl 1-$R_4$-4-$R_5$-pyrrole-2-acetate of the formula:

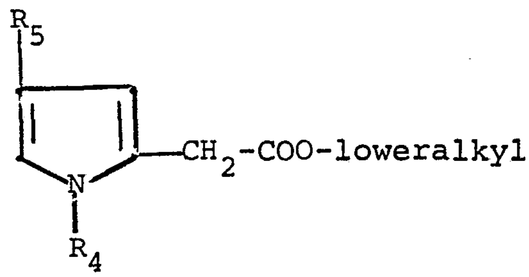

characterized by contacting a pyrrole reactant compound of formula:

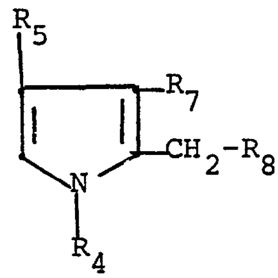

with a mixture of strong mineral acid, a loweralkanol and water, and recovering the resultant loweralkyl 1-$R_4$-4-$R_5$-pyrrole-2-acetate, wherein the foregoing formulas said $R_4$ and said $R_5$ each are independently selected from lower alkyl groups, and said $R_7$ and $R_8$ each are independently selected from -COOH, -COO-loweralkyl and -COOM, in which said M is an alkali metal selected from sodium and potassium.

2. The process of claim 1, characterized in that said acid is sulfuric acid.

3. The process of claim 1 or 2, characterized in that said lower-alkanol is ethanol.

4. The process of any one of claims 1 to 3, characterized in that said mixture constitutes from 3 to 15 moles of said acid per mole of said pyrrole reactant compound, from 0.5 to 6 moles of said acid per mole of said loweralkanol and from 0.3 to 10 moles of said acid per mole of water.

5. The process of any one of the preceding claims, characterized in that said contacting is carried out by addition of said mixture to said pyrrole reactant compound.

6. The process of any one of the preceding claims, characterized in that said recovering step comprises diluting the reaction mixture with water, extracting the dilute solution with an organic solvent and distilling the desired loweralkyl 1-$R_4$-4-$R_5$-pyrrole-2-acetate from said solvent.

7. The process of claim 6, characterized in that said organic solvent is methylene chloride or toluene.

8. The process of any one of the preceding claims, characterized in that said $R_7$ and $R_8$ are -COO-loweralkyl.

9. The process of claim 8, characterized in that the composition of said mixture ranges from 2 to 5 moles of said acid per mole of water and from 1 to 5 moles of said acid per mole of said alkanol.

10. The process of any one of claims 1-7, characterized in that said $R_7$ and $R_8$ are -COOH.

11. The process of claim 10, characterized in that the composition of said mixture ranges from 2 to 5 moles of said acid per mole of water and from 0.75 to 2 moles of said acid per mole of said alkanol.

12. The process of any one of the preceding claims, characterized in that said $R_4$ and $R_5$ are methyl.

0051442

European Patent Office

# EUROPEAN SEARCH REPORT

Application number

EP 81 30 5101.8

| Category | Citation of document with indication, where appropriate, of relevant passages | Relevant to claim | CLASSIFICATION OF THE APPLICATION (Int. Cl.³) |
|---|---|---|---|
| D | US - A - 3 752 826 (J.R. CARSON)<br>* column 8, lines 8 to 14, 30; example CXXV *<br>-- | 1,8, 10,12 | C 07 D 207/327 |
| D | US - A - 3 865 840 (J.R. CARSON)<br>* column 8, lines 59 to 64; column 9, line 25; example CXXV *<br>-- | 1,8, 10,12 | |
| | US - A - 3 428 648 (S. UMIO et al.)<br>* claims 20,21; examples 10,13 *<br>-- | 1,2,6, 10 | TECHNICAL FIELDS SEARCHED (Int. Cl.³) |
| | JOURNAL OF MEDICINAL CHEMISTRY<br>Vol. 16, No. 2, 1973<br>Washington D.C.<br>J.R. CARSON et al. "5-Benzoyl-1-methyl-pyrrole-2-acetic Acids as Antiinflammatory Agents. 2. The 4-Methyl Compounds"<br>pages 172 to 174<br>* page 173, scheme 1, formula VIII, IX; experimental section, compound IX *<br>-- | 1,6,8 | C 07 D 207/32 |
| A | LIEBIGS ANNALEN DER CHEMIE, Vol. 739<br>1970<br>A. TREIBS et al. "Eliminierungsreaktionen in der Pyrrol-Reihe"<br>pages 225 to 227<br>* complete document *<br>---- | | CATEGORY OF CITED DOCUMENTS<br>X: particularly relevant<br>A: technological background<br>O: non-written disclosure<br>P: intermediate document<br>T: theory or principle underlying the invention<br>E: conflicting application<br>D: document cited in the application<br>L: citation for other reasons |
| X | The present search report has been drawn up for all claims | | &: member of the same patent family, corresponding document |

| Place of search | Date of completion of the search | Examiner |
|---|---|---|
| Berlin | 11-01-1982 | BERTRAM |